(19) Europäisches Patentamt / European Patent Office / Office européen des brevets

(11) **EP 2 014 727 B1**

(12) **EUROPÄISCHE PATENTSCHRIFT**

(45) Veröffentlichungstag und Bekanntmachung des Hinweises auf die Patenterteilung:
**14.10.2009 Patentblatt 2009/42**

(51) Int Cl.:
***C09D 7/12*** *(2006.01)*   ***C09D 201/00*** *(2006.01)*
***G01N 33/558*** *(2006.01)*   ***G01N 33/543*** *(2006.01)*
***C12Q 1/00*** *(2006.01)*

(21) Anmeldenummer: **08157390.9**

(22) Anmeldetag: **02.06.2008**

(54) **Hydrophiler Beschichtungslack**

Hydrophilic coating finish

Revêtement hydrophile

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **07.06.2007 DE 102007026998**

(43) Veröffentlichungstag der Anmeldung:
**14.01.2009 Patentblatt 2009/03**

(73) Patentinhaber: **TESA SE**
**20253 Hamburg (DE)**

(72) Erfinder:
• **Neubert, Dr. Ingo**
**22850 Norderstedt (DE)**
• **Kampers, Maren**
**21217 Seevetal (DE)**

(56) Entgegenhaltungen:
**EP-A- 1 647 568**   **WO-A-20/05111606**
**WO-A-20/08006701**

Bemerkungen:
Die Akte enthält technische Angaben, die nach dem Eingang der Anmeldung eingereicht wurden und die nicht in dieser Patentschrift enthalten sind.

**Beschreibung**

Die vorliegende Erfindung betrifft einen hydrophilen Beschichtungslack, der nach Trocknung ein dauerhaftes schnelles Spreiten beziehungsweise einen dauerhaften schnellen Transport von biologischen Flüssigkeiten wie beispielsweise Blut, Urin, Speichel oder Zellflüssigkeit in Biosensoren oder analytischen Teststreifen ermöglicht und der vorzugsweise in einem Druckverfahren aufgebracht wird.

In der modernen Medizindiagnostik werden eine immer größere Anzahl analytischer Teststreifen, so genannte Diagnoseteststreifen, und Biosensoren verwendet. Hierzu zählen auch so genannte Microfluidic Devices und Lab-on-a-Chip-Anwendungen. Mit Hilfe der Biosensoren können zum Beispiel biologische Flüssigkeiten zum einen auf Krankheitserreger, Unverträglichkeiten, DNA- oder Enzym-Aktivität und zum anderen auf Gehalt an Glukose, Cholesterol, Proteinen, Ketonen, Phenylalanin oder Enzymen untersucht werden.

Auf den Biosensoren oder Teststreifen finden Nachweisreaktionen oder Reaktionskaskaden statt. Dazu muss die biologische Testflüssigkeit zu dem Reaktionsort beziehungsweise unterschiedlichen Reaktionsorten transportiert werden. Die modernen Biosensoren und Teststreifen bestehen daher aus zumindest einem Mikrokanal oder einem Mikrokanalsystem, durch das die Testflüssigkeit transportiert wird. Die Mikrokanäle haben typischerweise eine Höhe und Breite von 5 bis 1500 μm. Der Transport innerhalb der Kanäle erfolgt durch Kapillar- oder Zentrifugalkräfte. Die Ergebnisse der Nachweisreaktionen werden zumeist optisch oder elektrochemisch ausgelesen.

Für typische Anwendungen seien beispielhaft Microfluidic Devices (zum Beispiel US 2002/0112961 A1, US 6,601,613 B2, US 7,125,711 B2, EP 1 525 916 A1), Biosensoren (zum Beispiel DE 102 34 564 A1, US 5,759,364 A1) und Blutzuckerteststreifen (zum Beispiel WO 2005/033698 A1, US 5,997,817 A1) erwähnt. Weitere beispielhafte Anwendungen sind DNA-Microarrays und Immunoassays.

Bei all diesen Systemen ist es wichtig, den Flüssigkeitstransport durch die Kapillargeometrie und die Oberflächeneigenschaften der Kapillarinnenwandung zu steuern. Die Oberflächeneigenschaften können durch eine Beschichtung beeinflusst werden. Die Verwendung von Standardmaterialien ohne spezielle Beschichtung ist aufgrund der inhomogenen Oberflächeneigenschaften sowie der geringen Benetzbarkeit oft nicht geeignet. Eine aktuelle Entwicklungsrichtung bei Biosensoren und Teststreifen zielt auf die Verkürzung der Messzeit, was unter anderem eine Verkürzung der Transportgeschwindigkeit der biologischen Flüssigkeit erfordert.

In der Literatur sind verschiedene Untersuchungen zu den Themen Kapillarität und Transport von Flüssigkeiten in Kapillaren zu finden. Der Kapillardruck beziehungsweise die Steighöhe einer Flüssigkeitssäule in einer Kapillare sind abhängig von der Oberflächenspannung der Flüssigkeit, der Viskosität der Flüssigkeit, dem Benetzungswinkel und dem Kapillardurchmesser. Die Steighöhe wird nach folgender Formel bestimmt (Gleichung 1 (Gl. 1)):

$$h = \frac{4 * \gamma_l * \cos\theta}{g\,(\vartheta_l - \vartheta_g) * d} \qquad\qquad \text{Gl. 1}$$

h -     Steighöhe beziehungsweise Depressionshöhe
$\gamma_l$ -     Oberflächenspannung der Flüssigkeit
$\vartheta_l$     Dichte der Flüssigkeit
$\vartheta_g$ -     Dichte des Gases (Luft)
g     - Erdbeschleunigung
$\theta$ -     Kontaktwinkel (Benetzungswinkel, Randwinkel)
d     - Innendurchmesser der Kapillare

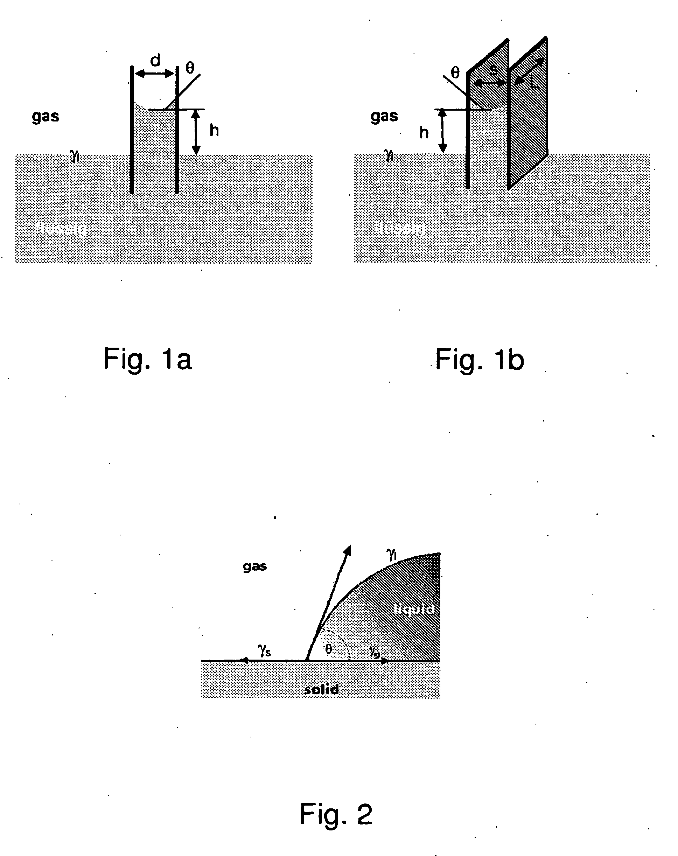

Figur 1 a veranschaulicht die Gleichung 1.

Bei der Betrachtung einer Kapillare, die aus zwei parallelen Platten besteht, wobei die Breite der Platten L deutlich größer als der Abstand d der Platten zueinander, vereinfacht sich die Gleichung 1 zu (bei s « L):

$$h \approx \frac{2 * \gamma_l * \cos\theta}{g * \vartheta_l * s} \qquad\qquad \text{Gl. 2}$$

h -     Steighöhe beziehungsweise Depressionshöhe

γ$_l$ - Oberflächenspannung der Flüssigkeit

$\vartheta_l$ -Dichte der Flüssigkeit

g - Erdbeschleunigung

θ - Kontaktwinkel (Benetzungswinkel, Randwinkel)

s - Abstand der parallelen Platten

Figur 1b veranschaulicht die Gleichung 1.

Aus Gleichung 1 und 2 ergibt sich, dass die Kapillarkräfte mit kleinerem Kapillardurchmesser beziehungsweise Plattenabstand zunehmen. Eine Verringerung der Flussgeschwindigkeit in einer Kapillare kann daher durch eine Vergrößerung des Querschnitts eines Mikrokanals erreicht werden. Ein weiterer wichtiger Parameter bei der Beeinflussung der Flussgeschwindigkeit einer gegebenen Flüssigkeit ist die Oberflächenspannung der Kanalinnenseite, wohingegen bei einer vorgegebenen Flüssigkeit die Viskosität als Parameter nicht variiert werden kann.

Im Falle eines sehr kleinen Benetzungswinkels zwischen Flüssigkeit und Kapillarwand kommt es zu einer Kapillar-Aszension, das heißt, die Flüssigkeit steigt in der Kapillare. Bei einem Kontaktwinkel von > 90° kommt es zu einer Kapillar-Depression, die Flüssigkeit wird aus der Kapillare herausgedrängt (W. Bohl "Technische Strömungslehre", 13., überarbeitete und erweiterte Auflage, Vogel Verlag, Juni 2005, ISBN: 3834330299, Seite 37f).

In der Literatur gibt es zahlreiche Untersuchungen zur Oberflächenspannung und zum Phänomen der Benetzbarkeit von Festkörpern.

Die Benetzung eines Festkörpers durch eine Flüssigkeit wird durch die Youngsche Gleichung (Gl. 3) beschrieben (siehe hierzu Figur 2):

$$\gamma_l \cdot \cos \theta = \gamma_s - \gamma_{sl} \qquad\qquad \text{Gl. 3}$$

θ - Kontaktwinkel (Benetzungswinkel, Randwinkel)

γ$_l$ - Oberflächenspannung der Flüssigkeit

γ$_s$ - Oberflächenspannung des Festkörpers

γ$_{sl}$ - Grenzflächenspannung zwischen der Flüssigkeit und dem Festkörper

Im Fall, dass die Oberflächenspannungen des Festkörpers und der Flüssigkeit deutlich unterschiedlich sind, erhält man einen Kontaktwinkel θ > 90°. Die Festkörperoberfläche ist durch die Flüssigkeit nicht benetzbar. Im Bereich von 90° bis 20° kommt es zu einer Benetzung der Festkörperoberfläche. Bei Kontaktwinkeln θ < 20° sind die Oberflächenspannungen zwischen Flüssigkeit und Festkörper sehr ähnlich, und es kommt zu einer sehr guten Benetzung der Festkörperoberfläche durch die Flüssigkeit. Bei Kontaktwinkeln θ « 20° (θ ~ 0°) spreitet die Flüssigkeit auf der Festkörperoberfläche aus (siehe ,,Die Tenside", Kosswig/Stache, Carl Hanser Verlag, 1993, ISBN 3-446-16201-1, Seite 23).

In der Literatur wird die Verwendung von Tensiden, dem Fachmann als grenzflächenaktive Substanzen bekannt, zur Verbesserung der Benetzbarkeit beschrieben. Tenside sind Moleküle oder Polymere, die aus einem unpolaren/hydrophoben Teil (Schwanz) und einer polaren/hydrophilen Gruppe (Kopf) bestehen. Zur Verbesserung der Benetzbarkeit von Oberflächen werden die Tenside der wässrigen Flüssigkeit zugegeben. Das Tensid bewirkt eine Herabsetzung der Oberflächenspannung der wässrigen Flüssigkeit an den Grenzflächen (flüssig-fest und flüssig-gasförmig). Dieser Effekt der Verbesserung der Benetzbarkeit der Oberflächen ist messbar in einer Verringerung des Kontaktwinkels und in der Verringerung der Oberflächenspannung der Flüssigkeit. Der Fachmann unterscheidet zwischen anionischen, kationischen, amphoteren und nichtionischen Tensiden. Der hydrophobe Schwanz von Tensiden kann aus linearen oder verzweigten Alkyl-, Alkylbenzyl-, perfluorierten Alkyl- oder Siloxan-Gruppen bestehen. Mögliche hydrophile Kopfgruppen sind anionische Salze von Carbonsäuren, Phosphorsäuren, Phosphonsäuren, Sulfaten, Sulfonsäuren oder Bernsteinsäureestern, kationische Ammoniumsalze oder nichtionische Polyglykoside, Polyamine, Polyglykolester, Polyglykolether, Polyglykolamine, polyfunktionelle Alkohole oder Alkoholethoxylate (siehe auch Ullmann's Encyclopedia of Industrial Chemistry, Vol. A25, 1994, Seite 747).

Prinzipiell kann eine Erhöhung der Oberflächenspannung beziehungsweise eine bessere Benetzbarkeit der Innenseite der Mikrokanäle zu einer Erhöhung der Transportgeschwindigkeit der biologischen Flüssigkeit innerhalb der Mikrokanäle führen. Durch hydrophile Beschichtungen mit polaren Polymeren wie zum Beispiel Polyvinylpyrrolidon, Polycaprolactam, Polyethylenglykol, Poly(meth)acrylsäure oder Polyvinylalkohol beziehungsweise Copolymeren mit entsprechenden polaren funktionellen Gruppen können besagte Erhöhung der Oberflächenspannung und damit bessere Benetzbarkeit erreicht werden. Die Benetzbarkeit beziehungsweise Hydrophilie dieser Beschichtungen sind jedoch für den schnellen Transport der biologischen Flüssigkeiten in den Mikrokanälen oft nicht ausreichend.

Ebenso ungeeignet sind chemische oder physikalische Modifikationen der Oberflächen. Standardmethoden hierfür sind Corona- und Flammbehandlung. Diese Behandlungen sind über die Zeit nicht stabil. Die durch die Oberflächenbehandlung deutlich erhöhte Oberflächenenergie verringert sich bereits nach wenigen Tagen auf den ursprünglichen Wert. Eine chemische Modifikation kann durch Ätzen der Oberfläche mit einer starken Säure erreicht werden. Zur Oberflächenätzung von technischen Folien werden zum Beispiel oxidierende Säuren wie Chromschwefelsäure oder Kaliumpermanganat in Verbindung mit Schwefelsäure verwendet. Polyesterfolien (PET) werden in der Technik üblicherweise durch chemische Behandlung mit zum Beispiel Trichloressigsäure oder Kaliumhydroxid an der Oberfläche hydrolysiert, wie es in der WO2005/11160 A1 offenbart ist. Bei diesen Methoden sind die Benetzbarkeit beziehungsweise die Oberflächenspannung auch nach Lagerung stabil. Allerdings sind die Benetzungseigenschaften über die Fläche inhomogen.

Die Oberfläche kann des Weiteren durch eine Plasmabehandlung modifiziert werden. Hierzu wird die Oberfläche im Vakuum mit einem Plasma behandelt. Durch die Einbringung von Gasen oder organischen Substanzen können die Oberflächeneigenschaften gezielt eingestellt werden. So lassen sich sowohl hydrophile als auch hydrophobe Schichten auf der Oberfläche erzeugen. Die Anwendung dieses Verfahrens wird in US 6,955,738 B2 beschrieben.

In WO 01/02093 A2 wird die Verwendung einer Folie mit einer mikrostrukturierten Oberfläche zur Kontrolle des Flüssigkeitstransportes beschrieben.

In DE 102 34 564 A1 wird ein Biosensor beschrieben, der aus einem planaren Sensor oder Teststreifen und einem kompartimentierten Reaktions- und Messkammeraufsatz, der durch Prägung einer PVC-Folie hergestellt ist, besteht. Der Probenaufnahmekanal und die Messkammer werden für den Transport der biologischen Flüssigkeit mit einem hydrophilen Gewebe oder einem Tensid ausgerüstet. Ein sehr ähnlicher elektrochemischer Sensor wird in US 5,759,364 A1 beschrieben. Der Sensor besteht aus einer bedruckten Grundplatte und einer geprägten Deckfolie aus PET oder Polycarbonat. Der Messraum ist hier mit einem Polyurethan-Ionomer für einen beschleunigten Flüssigkeitstransport beschichtet.

In mehreren Veröffentlichungen wird die Verwendung von hydrophilen Materialien wie Geweben (DE 30 21 166 A1), Membranen (DE 198 49 008 A1) und Folien (EP 1 358 896 A1, WO 01/67099 A1) erwähnt, ohne jedoch die hydrophilen Beschichtungen näher zu charakterisieren.

In der DE 198 15 684 A1 wird ein analytisches Hilfsmittel bestehend aus einer kapillaraktiven Zone, einem Klebebandstanzling und einer kapillaraktiven Abdeckfolie beschrieben. Die kapillaraktive Abdeckfolie besitzt hydrophile Oberflächeneigenschaften, die durch Bedampfung der Abdeckfolie mit Aluminium und anschließender Oxidation erreicht werden.

In der US 2005/0084681 A1 wird eine Oberfläche mit einer hydrophilen Beschichtung offenbart. Diese Beschichtung besteht aus einem Tensid, vorzugsweise einem nichtionischem Alkoholethoxylat, und einem Stabilisator, vorzugsweise einem Alkylbenzylsulfonat.

In EP 1 647 568 A1 wird eine so genannte Anti-Fog-Beschichtung beschrieben. Diese Beschichtung wird auf eine Polyesterfolie aufgebracht und dient der Vermeidung von Wassertröpfchenbildung bei Lebensmittelverpackungen. Bei der Anti-Fog-Beschichtung handelt es sich um eine hydrophile Beschichtung aus einem anionischen Tensid, einem Polyvinylpyrrolidon als Matrixpolymer und Wasser.

Heute sind bereits Folien mit vollflächiger hydrophiler Beschichtung für die Anwendung in medizinischen Diagnosestreifen kommerziell erhältlich, zum Beispiel die Produkte 9962 und 9971 von 3M Inc., deren Verwendung in US 2002/0110486 A1 und EP 1 394 535 A1 gezeigt wird, und ARflow® 90128 und ARflow® 90469 von Adhesives Research Inc., deren Verwendung in US 5,997,817 A1 gezeigt wird.

In der Literatur sind ebenfalls einige Beispiele bekannt, in denen gezielt hydrophile und hydrophobe Bereiche zur Steuerung des Flüssigkeitstransports genutzt werden. In US 6,601,613 B2 wird der Flüssigkeitstransport durch die Kapillargeometrie gesteuert. Als Möglichkeit wird ebenfalls die Verwendung von Tensiden oder hydrophoben Polymeren erwähnt, jedoch nicht genauer beschrieben.

In US 6,969,166 B2 wird eine modifizierte Oberfläche mit zwei unterschiedlichen Kontaktwinkeln beschrieben. Die Modifizierung erfolgt durch Digitaldruck (Tintenstrahldruck) von hydrophoben Polymeren auf Basis von Fluor- oder Siliconpolymeren beziehungsweise von hydrophilen Polymeren. Die in den Beispielen angegebenen statischen Kontaktwinkel der hydrophilen Beschichtung betragen 75°.

Wie der Stand der Technik zeigt, ist zur Realisierung eines schnellen Transports von biologischen Testflüssigkeiten in einem Messkanal die Verwendung von oberflächenaktiven hydrophilen Beschichtungen notwendig. Je nach verwendeten Rohstoffen sind hierbei erhebliche Nachteile bezüglich der Alterungsbeständigkeit und der Verträglichkeit der Rohstoffe untereinander zu beobachten. Diese Wechselwirkungen werden unter anderem von Popescu et al in "Untersuchung über die Wechselwirkung Polymer - Tensid" in Colloid & Polymer Science, Springer Verlag, Vol. 250, 1972, Seite 303f beschrieben.

Aufgabe der vorliegenden Erfindung ist, einen Beschichtungslack zur Verfügung zu stellen, der nach Entfernung des Lösemittels hydrophile Eigenschaften aufweist. Der getrocknete Beschichtungslack entspricht den Anforderungen für den Einsatz in Biosensoren und Diagnoseteststreifen und ist zum Aufbau derselben geeignet und ermöglicht im Speziellen einen Transport der biologischen Flüssigkeit in den Messkanälen. Hierbei ist weiterhin zu gewährleisten, dass die Eigenschaften und im Speziellen die Benetzungs- und Transporteigenschaften der sich nach der Trocknung des Beschichtungslacks ergebende Oberfläche auch nach einer langen Lagerzeit erhalten bleiben.

Gelöst wird diese Aufgabe durch einen Beschichtungslack, wie er im Hauptanspruch niedergelegt ist. Gegenstand der Unteransprüche sind vorteilhafte Weiterentwicklungen des Erfindungsgegenstandes. Des Weiteren umfasst die Erfindung die Verwendungsmöglichkeit des erfindungsgemäßen Beschichtungslacks wie in den Ansprüchen 18 bis 20 definiert wird, nach dessen Trocknung unter anderem in medizinischen Sensoren oder Diagnosestreifen zur Untersuchung von biologischen Flüssigkeiten.

Demgemäß betrifft die Erfindung einen Beschichtungslack insbesondere zur Verwendung in Biosensoren und Diagnosestreifen aus

mindestens einer oberflächenaktiven Substanz mit

- einer kritischen Mizell-Bildungskonzentration (CMC, 25°C) von höchstens 2,3 g/L und
- einer Oberflächenspannung (25 °C) der 0,1 Gew.-%igen wässrigen Lösung von höchstens 50 mN/m,

mindestens einem Lösemittel,
mindestens einem Bindemittel sowie
gegebenenfalls weiteren Additiven,
wobei die sich nach der Trocknung des Beschichtungslacks ergebende Oberfläche

- einen Kontaktwinkel mit Wasser von höchstens 30° und
- eine Oberflächenspannung von mindestens 60 mN/m

aufweist.

In einer vorteilhaften Ausführungsform der Erfindung weist die oberflächenaktive Substanz eine kritische Mizell-Bildungskonzentration (CMC, 25 °C) von höchstens 1,5 g/L und/oder eine Oberflächenspannung der 0,1 Gew.-%igen wässrigen Lösung von höchstens 45 mN/m auf.

Als oberflächenaktive Substanzen, vom Fachmann als Tenside bezeichnet, können Verbindungen aus linearen oder verzweigten Alkyl-, Alkylbenzyl-, perfluorierten Alkyl-oder Siloxan-Gruppen mit hydrophilen Kopfgruppen wie anionischen Salzen von Carbonsäuren, Phosphorsäuren, Phosphonsäuren, Sulfaten, Sulfonsäuren oder Sulfobernsteinsäure, wie kationischen Ammoniumsalzen oder wie nichtionischen Polyglykosiden, Polyaminen, Polyglykolestern, Polyglycolethern, Polyglykolaminen, polyfunktionellen Alkoholen oder Alkoholethoxylaten verwendet werden. Diese Auswahl ist eine beispielhafte Aufzählung und stellt keine Einschränkung des erfinderischen Gedankens auf die genannten Tenside dar.

Bei der Auswahl der oberflächenaktiven Substanz ist eine geringe kritische Mizell-Bildungskonzentration (CMC) von höchstens 2,3 g/L, vorzugsweise von höchstens 1,5 g/L und eine geringe Oberflächenspannung der 0,1 Gew.-%igen wässrigen Lösung von höchstens 50 mN/m, vorzugsweise von höchstens 45 mN/m notwendig, um einen schnellen Transport der biologischen Testflüssigkeit zu ermöglichen. Sowohl die CMC als auch die Oberflächenspannung sind ein Maß für die Aktivität beziehungsweise Wirksamkeit von Tensiden.

Überaschenderweise zeichnen sich diese Tenside besonders durch ihre herausragende Alterungsbeständigkeit aus.

Beispielhaft seien folgende geeignete Tenside genannt:

- nichtionische Fettalkoholethoxylate-Tenside zum Beispiel Tego Surten® W111 von Degussa AG oder Triton® X-100 und Tergitol® 15-S von Dow Chemicals Inc (CMC 1,0 g/L, Oberflächenspannung der 0,1 Gew.-%igen wässrigen Lösung 30 mN/m)
- nichtionische Fluortenside zum Beispiel Fluorad® FC-4430 und FC-4432 von 3M Inc., Zonyl® FSO-100 von DuPont Inc. und Licowet® F 40 von Clariant AG (CMC 0,1 g/L, Oberflächenspannung der 0,1 Gew.-%igen wässrigen Lösung 20 mN/m)
- nichtionische Silicontenside zum Beispiel Q2-5211 und Sylgard® 309 von Dow Corning Inc., Lambent® 703 von Lambent Technologie Inc. und Tegopren® 5840 von Degussa AG (CMC 0,2 g/L, Oberflächenspannung der 0,1 Gew.-%igen wässrigen Lösung 25 mN/m)
- ionisches Sulfobernsteinsäuresalze zum Beispiel Lutensit® A-BO von BASF AG, Aerosol® OT-NV von Cytec Industries Inc. oder Rewopol® SB DO 75 von Goldschmidt GmbH (CMC 1,5 g/L, Oberflächenspannung der 0,1 Gew.-%igen wässrigen Lösung 40 mN/m)

Vorzugsweise enthält der erfindungsgemäße Beschichtungslack als oberflächenaktive Substanz ein anionisches Tensid und besonders bevorzugt Sulfobernsteinsäuresalze.

Der erfindungsgemäße Beschichtungslack enthält mindestens ein Lösemittel, das nach der Applikation des Lacks entfernt wird, so dass eine feste, trockene und durch Kontakt mit festen Gegenständen wie zum Beispiel Walzen oder Kunststoffmaterialien nicht übertragbare Beschichtung entsteht. Als Lösemittel werden Wasser, Alkohole, Ethanol, oder höhersiedende Alkohole wie n-Butanol oder Ethoxyethanol, Ketone wie Butanon, Ester wie Essigsäureethylester, Alkane wie Hexan, Toluol oder Gemische aus den vorher genannten Lösemitteln eingesetzt. Vorzugsweise werden polare organische Lösemittel wie zum Beispiel Alkohole und besonders bevorzugt Wasser verwendet. Unter den Alkoholen sind weiterhin Ethanol, Isopropanol oder Butanol bevorzugt.

Der erfindungsgemäße Beschichtungslack enthält zumindest ein Bindemittel. Das Bindemittel dient zur Einstellung der Viskosität des Beschichtungslacks. Eine Anpassung der Viskosität ist je nach verwendetem Beschichtungsprozess notwendig. In der folgenden Tabelle sind beispielhaft typische Viskositäten für unterschiedliche Beschichtungsverfahren aufgezählt.

| Beschichtungsverfahren | Erforderliche dynamische Viskosität des Beschichtungslacks für das jeweilige Beschichtungsverfahren [mPa*s] |
|---|---|
| Rakelbeschichtung | 500 - 40.000 |
| Rasterwalzenauftrag | 1 - 5.000 |
| Spray-Coating | 1 - 100 |
| Mayer-Bar-Beschichtung | 20 - 1.000 |
| Flexodruck | 50 - 500 |
| Tintenstrahldruck | 1 - 30 |
| Siebdruck | 500 - 50.000 |
| Offsetdruck | 40.000 - 100.000 |
| Tiefdruck | 50 - 200 |

Für den erfindungsgemäßen Beschichtungslack können alle aus der Druckfarbenindustrie bekannten filmbildenden Bindemittel verwendet werden.

Vorzugsweise werden als Bindemittel Polymere oder Copolymere mit Carboxyl-, Carboxylat-, Amin-, Ammonium-, Amid- oder Alkohol-Funktionalitäten und besonders bevorzugt entsprechende wasserlösliche Polymere oder Copolymere verwendet.

Beispielhaft und nicht einschränkend seien als geeignete Binder Homo- oder Copolymere wie Polyvinylpyrrolidon, Polyvinylbutyral, Polyester, Polyacrylat, Poly(meth)acrylsäure, Polyvinylacetat, teilweise hydrolysiertes Polyvinylacetat, Polyvinylalkohol, Poly(meth)acrylamid, Polyamid, Polyethylenglykol, Polypropylenglykol, CelluloseDerivate genannt. Die genannten Polymere können auch als Dispersionen eingesetzt werden.

In einer bevorzugten Variante des erfindungsgemäßen Beschichtungslacks wird ein Polyvinylalkohol als Binder eingesetzt. Polyvinylalkohole werden aus Polyvinylacetat durch Hydrolyse der Acetat-Funktionalität hergestellt. Die Eigenschaften der Polyvinylalkohole können zum einen über das Molekulargewicht des Polymers und zum anderen über den Hydrolysegrad gesteuert werden. Bevorzugt wird ein Polyvinylalkohol mit einem Hydrolysegrad von > 85 Mol-% und besonders bevorzugt von > 95 Mol-% verwendet. Beispielhaft für diese Polymerklasse seien Mowiol® von Kuraray oder Polyviol® von Wacker Chemie GmbH erwähnt.

In einer weiteren bevorzugten Variante des erfindungsgemäßen Beschichtungslacks wird ein Polyvinylbutyral als Binder eingesetzt. Polyvinylbutyral wird aus Polyvinylalkohol durch Veresterung mit n-Butyraldehyd erhalten. Die Eigenschaften werden durch das Molekulargewicht, den Hydrolysegrad und dem Acetalisierungsgrad bestimmt. Bevorzugt wird ein Polyvinylbutyral mit einem Vinylacetalgehalt von >75 Gew.-%, einem Vinylacetatgehalt von < 5 Gew.-% und einem Vinylalkoholgehalt von 15 bis 30 Gew.-% verwendet. Beispielhaft für diese Polymerklasse seien Mowital® von Kuraray oder Pioloform® von Wacker Chemie GmbH erwähnt.

In einer weiteren bevorzugten Variante des erfindungsgemäßen Beschichtungslacks wird ein Polyesterharz oder ein Maleinatharz mit freien Carboxyl- und Hydroxygruppen als Binder eingesetzt. Um eine Wasserlöslichkeit zu erreichen, wird das Polyesterharz mit einer Ammoniak-Lösung oder mit einem Amin neutralisiert. Beispiel für ein solches Harz sind Rokrapol® 7160 oder Erkamar® VP 4760 von Robert Kraemer GmbH.

In einer weiteren bevorzugten Variante des erfindungsgemäßen Beschichtungslacks wird ein Cellulose-Derivat als Binder eingesetzt. Besonders geeignet ist hierbei Carboxymethylcellulose und Celluloseacetat verwendet.

Der Beschichtungslack kann darüber hinaus ebenfalls organische Farbstoffe, Fluoreszenzfarbstoffe, anorganische Pigmente, Alterungsschutzmittel und/oder Füllstoffe enthalten (siehe hierzu "Plastics Additives Handbook", Kapitel "Antioxidants", "Colorants", "Fillers", Carl Hanser Verlag, 5. Auflage).

Diese Additive sind aber nicht zwingend erforderlich, der erfindungsgemäße Beschichtungslack erfüllt bereits ohne weitere Additive alle Anforderungen.

In einer bevorzugten Variante beträgt die dynamische Viskosität des erfindungsgemäßen Beschichtungslacks vor der Trocknung 50 bis 500 mPa*s.

Die sich nach der Trocknung des Beschichtungslacks ergebende Oberfläche weist vorteilhafterweise einen Kontaktwinkel mit Wasser von höchstens 23° und eine Oberflächenspannung von mindestens 65 mN/m auf.

Die Beschichtung beziehungsweise der Beschichtungslack nach deren beziehungsweise dessen Trocknung weist vorteilhafterweise eine Transportgeschwindigkeit von mindestens 20 mm/s und vorzugsweise mindestens 40 mm/s für wässrige Testflüssigkeit auf. Die Beschichtung zeichnet sich durch eine sehr gute Lagerstabilität aus. Das zeigt sich darin, dass sich die Transportgeschwindigkeit auch nach der Lagerung von 10 Wochen bei 70 °C nur um maximal 20 % und vorteilhafterweise um maximal 10% des Ursprungswerts verlangsamt.

Der erfindungsgemäße Beschichtungslack kann auf dem Fachmann geläufige und übliche Trägermaterialien wie Folien aus Polyethylen, Polypropylen, Polypropylen, Polyvinylchlorid, Polyester und besonders bevorzugt Polyethylenterephthalat (PET) vollflächig oder aber partiell beschichtet werden. Hierbei kann es sich um Monofolien, coextrudierte oder laminierte Folien handeln, die streckt, monoaxial oder biaxial versteckt sind. Die Oberfläche der Folien kann durch geeignete Verfahren wie zum Beispiel Prägen, Ätzen oder Lasern mikrostrukturiert sein.

Die Verwendung von Laminaten, Vliesen, Geweben oder Membranen als Träger ist ebenfalls möglich.

Diese Aufzählungen sind beispielhaft und nicht abschließend.

Die Trägermaterialien können zur besseren Verankerung der Beschichtung nach den Standardverfahren chemisch oder physikalisch vorbehandelt sein, beispielhaft seien Corona- oder die Flammenbehandlung genannt. Zur Haftvermittlung ist ebenfalls eine Primerung des Trägermaterials mit zum Beispiel PVC, PVDC, thermoplastischen Polyestercopolymeren, Polyurethanen möglich. Die Verankerung von Druckfarben wird üblicherweise nach einem Verankerungstest überprüft, wie er unter den Prüfmethoden dargelegt ist.

Die Dicke des Trägermaterials beträgt 12 bis 350 $\mu$m und vorzugsweise 50 bis 150 $\mu$m.

Für die Applikation können die üblichen Beschichtungsverfahren wie beispielsweise Spray-Coating, Rasterwalzenauftrag, Mayer-Bar-Beschichtung, Mehrwalzenauftragsbeschichtung oder Druckverfahren verwendet werden. Besonders bevorzugt ist die Applikation mittels eines Druckverfahrens wie Siebdruck, Flexodruck, Digitaldruck, Tintenstrahldruck oder Kondensationsbeschichtung. Bei der Auswahl des Druckverfahren beziehungsweise des Drucklackes sind die Parameter Viskosität, Druckgenauigkeit, Güte der Druckoberfläche und Druckgeschwindigkeit zu berücksichtigen. Bei den Druckverfahren Siebdruck, Digitaldruck und Tintenstrahldruck werden vorwiegend separate Rasterpunkte erzeugt. Hierdurch kann die Oberflächenspannung der Bereiche durch den Abstand und Größe der einzelnen Rasterpunkte gesteuert werden. Die bevorzugte Ausführung besteht jedoch in einer Applikation als geschlossener Film. Besonders bevorzugt erfolgt die Applikation des erfindungsgemäßen Beschichtungslacks partiell und mittels eines Druckverfahrens, besonders bevorzugt im Flexodruck.

Vorteilhaft lässt sich der erfindungsgemäße getrocknete Beschichtungslack in medizinischen Anwendungen, vorzugsweise in Diagnosestreifen, Biosensoren, Point-of-Care-Devices oder Microfluidic Devices, mittels derer biologische Flüssigkeiten untersucht werden, verwenden.

Besonders geeignet ist die Anwendung in Biosensoren oder analytischen Teststreifen, wobei die Beschichtung derart angeordnet ist, dass das beschichtete Trägermaterial eine Wand eines Transport- oder Reaktionskanals bildet.

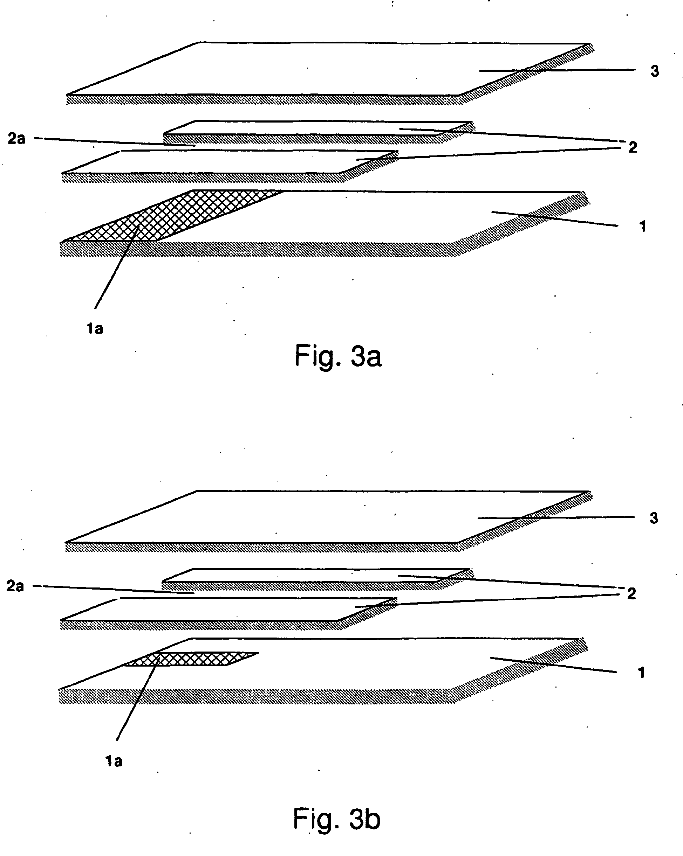

Die Figuren 3a und 3b zeigen einen beispielhaften Aufbau eines Diagnosestreifens mit einem Mikrokanal 2a, der in diesem Fall durch einen Stanzling eines doppelseitigen Haftklebebandes 2 gebildet wird. Der Mikrokanal 2a kann jedoch auch durch andere Verfahren wie zum Beispiel Mikroprägung, Spritzguss, Laserverfahren, lithographische Verfahren oder Sandblasen hergestellt werden. Der aus dem Haftklebeband 2 gebildete Mikrokanal 2a ist einseitig mit der Basisfolie 3 verklebt. Diese Basisfolie 3 bildet somit eine Wandung des Mikrokanals und kann zusätzlich Funktionsschichten wie zum Beispiel elektrische Leiterbahnen oder Schichten mit Nachweisreagenzien oder Enzymen aufweisen. Der Mikrokanal 2a wird als weitere Wandung mit einer Folie 1, die zumindest partiell mit dem erfindungsgemäßen Beschichtungslack 1a beschichtet ist, wobei die Beschichtung zur Innenseite des Mikrokanals zeigt, verschlossen. Die Beschichtung 1a, die zum Beispiel streifenförmig wie in Figur 3a oder partiell wie in Figur 3b aufgetragen ist, befindet sich hierbei an der Öffnung des Mikrokanals 2a, um den Start des Flüssigkeitstransports in den Kanal zu ermöglichen.

Bei der Verwendung des erfindungsgemäßen Beschichtungslacks nach dessen Trocknung als Abdeckung beziehungsweise als Wandung eines Transport- oder Reaktionskanals in Biosensoren oder analytischen Teststreifen erfolgt der Transport der Testflüssigkeit überraschender Weise sehr effektiv und zuverlässig.

Für den Fachmann überraschend ist, dass in einem wie in Figur 3a dargestellten Kanal der Flüssigkeitstransport durch die Beschichtung mit dem erfindungsgemäßen Beschichtungslack erheblich beschleunigt werden kann. Die Beschichtung zeichnet sich durch eine herausragende Alterungsstabilität aus. Der Beschichtungslack kann hierbei mittels eines geeigneten Verfahrens wie zum Beispiel einem Druckverfahren Punktgenau an die Stelle appliziert werden, an der ein schneller Flüssigkeitstransport benötigt wird (Figur 3b). Besonders überraschend und nicht vorhersehbar ist, dass das verwendete Bindemittel weder die Aktivität der oberflächenaktiven Substanz verringert, noch die Alterungsbeständigkeit verschlechtert oder die Nachweisreaktion auf den Teststreifen beeinflusst.

**Prüfmethoden**

**Oberflächenspannung und Kontaktwinkelmessung**

Die Messung des Kontaktwinkels mit Wasser und der Oberflächenspannung auf festen Oberflächen erfolgt nach EN 828:1997 mit einem Gerät G2/G402 der Firma Krüss GmbH. Die Oberflächenspannung wird mit der Methode nach Owens-Wendt-Rabel&Kaeble nach Messung des Kontaktwinkels mit deionisiertem Wasser und Diiodomethan bestimmt. Die Werte ergeben sich jeweils aus der Mittlung von vier Messwerten.

**Statische Oberflächenspannung (Flüssigkeit) und CMC**

Die Messung der Oberflächenspannung von wässrigen Flüssigkeiten erfolgt nach der Ringmethode (du Nouy) nach DIN EN 14210 mit dem Gerät Tensiometer K100 der Firma Krüss GmbH bei 20 °C.
Die kritische Micell-Bildungskonzentration (CMC) wird über die automatische Dosierung des Tensides durch das Tensiometer K100 der Firma Krüss GmbH und anschließender Auswertung des Konzentrations-Oberflächenverhalten bestimmt (DIN 53914).

**Dynamische Viskositätsmessung**

Die Viskositätsmessung des Beschichtungslacks wird mit einem Rheometrix DSR 200N bei einer Scherrate von 10 1/s mit einem Kegel-Platte-System mit einem Durchmesser von 50 mm durchgeführt.

**Kanaltest (Funktionstest)**

Zur Beurteilung des Transportverhaltens einer wässrigen Testflüssigkeit wird ein Kapillartest durchgeführt. Hierzu wird auf eine 100 $\mu$m Dicke PET-Folie (Hostaphan@ RN 100 von Mitsubishi Polyesterfilm GmbH) ein Stanzling eines doppelseitigen Klebebands mit der Dicke von 80 $\mu$m (tesa® 4980, ein doppelseitig klebendes Haftklebeband bestehend aus einer beidseitig mit einer Acrylatklebemasse (jeweils 34 g/m$^2$) beschichteten 12 $\mu$m PET-Trägerfolie, Produktdicke 80 $\mu$m) laminiert. Der Stanzling bildet einen Messkanal mit einer Breite von 1 mm und einer Länge von 3 cm. Der Kanal ist nach beiden Seiten offen. Dieser Messkanal wird anschließend mit der zu prüfenden Folie bedeckt, so dass die zu prüfende Oberfläche eine Wand des Kanals bildet. Dabei werden die Bereiche so positioniert, dass sich der hydrophile Bereich am Rand des Kanals befindet und der hydrophobe Bereich im Inneren des Kanals. Der Kanal beziehungsweise die Kapillare haben die Abmaße Höhe 75 $\mu$m und Breite 1 mm (siehe hierzu Figur 3a).
Der Testkanal wird mit der Öffnung, an dessen Ende der hydrophile Bereich positioniert ist, in eine Testflüssigkeit, bestehend aus deionisiertem Wasser und 1 Gew.-% Naphtholrot, gehalten. Der Transport der Testflüssigkeit im hydrophilen Bereich wird mittels einer Videokamera beobachtet und die Transportgeschwindigkeit ermittelt. Der Kanaltest wird auch nach einer Lagerung bei 23 °C, 40 °C und 70 °C der zu testenden Folien oder Kanäle durchgeführt, um die Alterungs- und Lagerungsbeständigkeit zu prüfen.
Als Testflüssigkeit werden ebenfalls biologische Flüssigkeiten wie Blut verwendet, diese sind allerdings weniger gut geeignet, da diese Eigenschaftsschwankungen unterliegen. So schwankt zum Beispiel die Viskosität von Blut sehr stark. Die Viskosität von Blut ist abhängig vom Hämatokrit-Wert.

**Verankerung**

Um die Verankerung der Beschichtung nach der Applikation und Trocknung auf einem Trägermaterial zu bestimmen, wird im so genannten tesa-Verankerungstest ein einseitiges Büroklebeband wie zum Beispiel tesa 4140 (23 g/m$^2$ Acylatdispersions-Klebmasse, 35 $\mu$m BOPP-Trägerfolie) über die Beschichtung geklebt und von Hand angedrückt. Das Klebeband wird anschließend mit einem Ruck abgezogen. Es wird nun beurteilt, ob die Beschichtung eine ausreichende Verankerung zum Substrat hat oder ob die Beschichtung mit dem Klebeband von dem Substrat teilweise oder vollflächig abgezogen wird.
Im Folgenden soll die Erfindung anhand mehrerer Beispiele näher erläutert werden, ohne damit die Erfindung unnötig einschränken zu wollen.

**Beispiele**

| Rohstoff | Hersteller | Rohstoffart | Eigenschaften |
|---|---|---|---|
| Lutensit A-BO | BASF AG | Na-düsoctylsulfosuccinat | Tensid<br>CMC 1,5 g/L<br>40 mN/m |
| Rewopol SB DO 75 | Degussa AG | Na-düsoctylsulfosuccinat | Tensid<br>CMC 1,5 g/L<br>40 mN/m |
| Tegopren W 5840 | Degussa AG | Siloxan-Ethoxylate | Tensid<br>CMC 0,05 g/L<br>23 mN/m |
| Mowiol 4-98 | Kuraray Specialities | Polyvinylalkohol | 98,4% Vinylalkohol<br>1,5% Vinylacetat |
| Piloform BM 18 | Wacker Chemie | Polyvinylbutyral | 18% Vinylalkohol<br>2% Vinylacetat<br>80% Vinylacetal |
| Luvitec K60 | BASF AG | Polyvinylpyrrolidon | K-Wert 60* |
| NeoRez R650 | Neoresins Inc. | PUR-Primer | |
| *: Die Messung des K-Werts erfolgt in 1 Gew.-%iger Lösung in Toluol bei 25 °C (DIN 53 726) | | | |

**Beispiel 1**

Es werden 10 kg Mowiol 4-98 und 0,3 kg Rewopol SB DO 75 in 60 kg Wasser unter ständigem Rühren gelöst. Der so entstandene Beschichtungslack hat eine Viskosität von 130 mPa*s. Der Beschichtungslack wird nun an einer Nilpeter-Druckmaschine im Flexodruck auf eine coronavorbehandelt PET-Folie (Hostaphan® RN 100 von Mitsubishi Polyesterfilm GmbH) gedruckt und mittels IR-Strahler getrocknet.

Der Beschichtungslack lässt sich sehr gut im Druckverfahren auf die PET-Folie übertragen. Die Beschichtung zeigt sehr gute Benetzungseigenschaften und eine hohe Geschwindigkeit beim Transport der wässrigen Testflüssigkeit im Testkanal. Die Transportgeschwindigkeit verlangsamt sich lediglich um 5% nach der Lagerung der Teststreifen für 10 Wochen bei 70 °C.

**Beispiel 2**

Der Beschichtungslack aus Beispiel 1 wird wie in Beispiel 1 auf eine PET-Folie gedruckt und getrocknet. Die PET-Folie ist jedoch zuvor mit dem Primer NeoRez R650 beschichtet worden.

Analog Beispiel 1 zeigt die hydrophile Beschichtung gleich gute Benetzungseigenschaften und eine hohe Transportgeschwindigkeit der wässrigen Testflüssigkeit im Testkanal. Diese Variante zeichnet sich durch eine sehr gute Verankerung der Beschichtung auf der PET-Folie aus.

**Beispiel 3**

Es werden 4 kg Mowiol 4-98 und 0,2 kg Tegopren W 5840 in 60 kg Wasser unter ständigem Rühren gelöst. Der so entstandene Beschichtungslack hat eine Viskosität von 27 mPa*s. Der Beschichtungslack wird im Tintenstrahldruck auf eine mit NeoRez R650 beschichtete PET-Folie (Hostaphan® RN 100 von Mitsubishi Polyesterfilm GmbH) gedruckt und mittels IR-Strahler getrocknet.

Der Beschichtungslack lässt sich sehr gut im Tintenstrahldruck auf die Folie applizieren. Die Benetzungseigenschaften und die Transportgeschwindigkeit der Beschichtung sind sehr gut. Nach der Lagerung fällt der Wert für die Transportgeschwindigkeit auf 65 % des Ursprungswertes zurück.

**Beispiel 4**

Es werden 12 kg Piloform BM 18 und 0,3 kg Lutensit A-BO in 60 kg Wasser unter ständigem Rühren gelöst.

Der so entstandene Beschichtungslack hat eine Viskosität von 180 mPa*s. Der Beschichtungslack wird nun an einer Nilpeter Druckmaschine im Flexodruck auf eine coronavorbehandelt PET-Folie (Hostaphan® RN 100 von Mitsubishi Polyesterfilm GmbH) gedruckt und mittels IR-Strahler getrocknet.

Die Beschichtung zeigt ebenfalls sehr gute Benetzungseigenschaften und eine hohe Transportgeschwindigkeit der wässrigen Testflüssigkeit im Testkanal. Diese Variante zeichnet sich durch eine sehr gute Verankerung der Beschichtung auf der PET-Folie aus.

Die Alterungsbeständigkeit der Eigenschaften ist ebenfalls gut.

| | Einheit | Beispiel 1 | Beispiel 2 | Beispiel 3 | Beispiel 4 |
|---|---|---|---|---|---|
| Kontakt-Winkel mit Wasser | ° | 20 | 19 | 5 | 23 |
| Oberflächenspannung | mN/m | 68 | 69 | 71 | 65 |
| Geschwindigkeit im Kanaltest | mm/s | 56 | 56 | 58 | 53 |
| Kanaltest nach Lagerung* | mm/s | 53 (-5%) | 54 (-4%) | 32 (-45%) | 50 (-6%) |
| Verankerung der Beschichtung | | o | ++ | ++ | ++ |
| * 10 Wochen bei 70 °C; -- schlecht, o mittelmäßig, ++ sehr gut | | | | | |

## Gegenbeispiele

### Gegenbeispiel 1

Es werden 10 kg Mowiol 4-98 in 60 kg Wasser unter ständigem Rühren gelöst. Der so entstandene Beschichtungslack hat eine Viskosität von 130 mPa*s. Der Beschichtungslack wird nun an einer Nilpeter-Druckmaschine im Flexodruck auf eine coronavorbehandelt PET-Folie (Hostaphan® RN 100 von Mitsubishi Polyesterfilm GmbH) gedruckt und mittels IR-Strahler getrocknet.

Die Beschichtung zeigt deutliche schlechtere Benetzungs- und Transporteigenschaften. Der Kanaltest zeigt, dass die Flüssigkeit nicht in den Kanal transportiert wird.

### Gegenbeispiel 2

Es werden 0,3 kg Rewopol SB DO 75 in 60 kg Wasser unter ständigem Rühren gelöst. Der so entstandene Beschichtungslack hat eine Viskosität von 1 mPa*s. Der Beschichtungslack wird versucht im Flexodruck auf eine coronavorbehandelt PET-Folie (Hostaphan® RN 100 von Mitsubishi Polyesterfilm GmbH) zu drucken. Das Drucken ist nicht möglich, da die Viskosität der Lösung zu gering ist. Des Weiteren ist es nicht möglich, einen geschlossenen Film mit der Beschichtung zu erhalten. Es können keine Messungen an der Beschichtung durchgeführt werden.

### Gegenbeispiel 3

Es werden 8 kg Luvitec K60 und 0,2 kg Lutensit A-BO in 60 kg Wasser unter ständigem Rühren gelöst. Der so entstandene Beschichtungslack hat eine Viskosität von 110 mPa*s. Der Beschichtungslack wird nun an einer Nilpeter-Druckmaschine im Flexodruck auf eine coronavorbehandelt PET-Folie (Hostaphan® RN 100 von Mitsubishi Polyesterfilm GmbH) gedruckt und mittels IR-Strahler getrocknet.

Die Beschichtung zeigt deutliche schlechtere Benetzungs- und Transporteigenschaften. Der Flüssigkeitstransport im Kanaltest ist sehr inhomogen und schwankt über einen weiten Bereich. In 50 % der Versuche findet kein Flüssigkeitstransport statt.

| | Einheit | Gegenbeispiel 1 | Gegenbeispiel 2 | Gegenbeispiel 3 |
|---|---|---|---|---|
| Kontakt-Winkel mit Wasser | ° | 37 | - | 33 |
| Oberflächenspannung | mN/m | 56 | - | 59 |
| Geschwindigkeit im Kanaltest | mm/s | - | - | 0 - 10 |
| Kanaltest nach Lagerung* | mm/s | - | - | - |

(fortgesetzt)

|  | Einheit | Gegenbeispiel 1 | Gegenbeispiel 2 | Gegenbeispiel 3 |
|---|---|---|---|---|
| Verankerung der Beschichtung |  | o | - | ++ |
| * 10 Wochen bei 70 °C; -- schlecht, o mittelmäßig, ++ sehr gut | | | | |

**Patentansprüche**

1. Beschichtungslack aus
   mindestens einer oberflächenaktiven Substanz mit

   - einer kritischen Mizell-Bildungskonzentration (CMC, 25 °C) von höchstens 2,3 g/L und
   - einer Oberflächenspannung (25 °C) der 0,1 Gew.-%igen wässrigen Lösung von höchstens 50 mN/m,

   mindestens einem Lösemittel,
   mindestens einem Bindemittel sowie
   gegebenenfalls weiteren Additiven,
   wobei die sich nach der Trocknung des Beschichtungslacks ergebende Oberfläche

   - einen Kontaktwinkel mit Wasser von höchstens 30° und
   - eine Oberflächenspannung von mindestens 60 mN/m

   aufweist.

2. Beschichtungslack nach Anspruch 1, **dadurch gekennzeichnet, dass**
   die oberflächenaktive Substanz eine kritische Mizell-Bildungskonzentration (CMC, 25°C) von höchstens 1,5 g/L und/oder eine Oberflächenspannung der 0,1 Gew.-%igen wässrigen Lösung von höchstens 45 mN/m aufweist.

3. Beschichtungslack nach einem der Ansprüche 1 oder 2, **dadurch gekennzeichnet, dass**
   die oberflächenaktive Substanz ein anionisches Tensid ist.

4. Beschichtungslack nach zumindest einem der Ansprüche 1 bis 3, **dadurch gekennzeichnet, dass**
   die oberflächenaktive Substanz ein Tensid auf Basis eines Sulfobernsteinsäuresalzes ist.

5. Beschichtungslack nach zumindest einem der vorherigen Ansprüche, **dadurch gekennzeichnet, dass**
   als Lösungsmittel mindestens ein polares Lösemittel verwendet wird.

6. Beschichtungslack nach zumindest einem der vorherigen Ansprüche, **dadurch gekennzeichnet, dass**
   als Lösungsmitten Alkohol verwendet wird.

7. Beschichtungslack nach zumindest einem der vorherigen Ansprüche, **dadurch gekennzeichnet, dass**
   als Lösungsmittel Wasser verwendet wird.

8. Beschichtungslack nach zumindest einem der vorherigen Ansprüche, **dadurch gekennzeichnet, dass**
   als Bindemittel mindestens ein Polymer oder ein Copolymer mit Carboxyl-, Carboxylat-, Amin-, Ammonium-, Amid- oder Alkohol-Funktionalitäten verwendet wird.

9. Beschichtungslack nach zumindest einem der vorherigen Ansprüche, **dadurch gekennzeichnet, dass**
   als Bindemittel ein wasserlösliches Polymer oder ein wasserlösliches Copolymer verwendet wird.

10. Beschichtungslack nach zumindest einem der vorherigen Ansprüche, **dadurch gekennzeichnet, dass**
    das Bindemittel gewählt ist aus der Gruppe Polyvinylalkohol, Polyvinylbutyral, Polyesterharz, Maleinatharz oder Cellulose-Derivat.

11. Beschichtungslack nach zumindest einem der vorherigen Ansprüche, **dadurch gekennzeichnet, dass**
    der Beschichtungslack vor der Trocknung eine Viskosität von 50 bis 500 mPa*s aufweist.

12. Beschichtungslack nach zumindest einem der vorherigen Ansprüche, **dadurch gekennzeichnet, dass** die sich nach der Trocknung des Beschichtungslacks ergebende Oberfläche

- einen Kontaktwinkel mit Wasser von höchstens 23° und
- eine Oberflächenspannung von mindestens 65 mN/m

aufweist.

13. Beschichtungslack nach zumindest einem der vorherigen Ansprüche, **dadurch gekennzeichnet, dass** die Transportgeschwindigkeit einer wässrigen Testflüssigkeit (deionisiertes Wasser und 1 Gew.-% Naphtholrot) in einem Kanal (75 $\mu$m x 1mm), in der der getrocknete Beschichtungslack eine Wand des Kanals bildet, mindestens 20 mm/s beträgt.

14. Beschichtungslack nach zumindest einem der vorherigen Ansprüche, **dadurch gekennzeichnet, dass** die Transportgeschwindigkeit einer wässrigen Testflüssigkeit (deionisiertes Wasser und 1 Gew.-% Naphtholrot) in einem Kanal (75 $\mu$m x 1mm), in der der getrocknete Beschichtungslack eine Wand des Kanals bildet, mindestens 40 mm/s beträgt.

15. Beschichtungslack nach zumindest einem der vorherigen Ansprüche, **dadurch gekennzeichnet, dass** sich die Trartsportgeschwindigkeit einer wässrigen Testflüssigkeit (deionisiertes Wasser und 1 Gew.-% Naphtholrot) in einem Kanal (75 $\mu$m x 1mm), in der der getrocknete Beschichtungslack eine Wand des Kanals bildet, nach der Lagerung von 10 Wochen bei 70 °C um maximal 20 % des Ursprungswerts verlangsamt.

16. Beschichtungslack nach zumindest einem der vorherigen Ansprüche, **dadurch gekennzeichnet, dass** der Beschichtungslack mittels eines Druckverfahrens auf ein Trägermaterial aufgebracht wird.

17. Beschichtungslack nach zumindest einem der vorherigen Ansprüche, **dadurch gekennzeichnet, dass** der Beschichtungslack im Flexodruckverfahren auf ein Trägermaterial aufgebracht wird.

18. Verwendung des getrockneten Beschichtungslacks nach zumindest einem der vorherigen Ansprüche als Beschichtung in medizinischen Anwendungen.

19. Verwendung des getrockneten Beschichtungslacks nach zumindest einem der vorherigen Ansprüche als Beschichtung in Diagnosestreifen, Biosensoren, Point-of-Care-Devices oder Microfluidic Devices, mittels derer biologische Flüssigkeiten untersucht werden.

20. Verwendung des getrockneten Beschichtungslacks nach zumindest einem der vorherigen Ansprüche als Beschichtung in Biosensoren oder analytischen Teststreifen, wobei das Material derart angeordnet ist, dass die Beschichtung zumindest partiell mindestens eine Wand eines Transport- oder Reaktionskanals bildet.

**Claims**

1. Coating lacquer composed of
at least one surfactant substance with

- a critical micelle-formation concentration (CMC, 25°C) of at most 2.3 g/L and
- a surface tension (25°C) of its 0.1% strength by weight aqueous solution of at most 50 mN/m,

at least one solvent,
at least one binder and also
if appropriate, further additives,
where the surface obtained after drying of the coating lacquer has

- a contact angle with water of at most 30° and
- a surface tension of at least 60 mN/m.

2. Coating lacquer according to Claim 1,

**characterized in that**
the surfactant substance has a critical micelle-formation concentration (CMC, 25°C) of at most 1.5 g/L and/or a surface tension of its 0.1% strength by weight aqueous solution of at most 45 mN/m.

3. Coating lacquer according to Claim 1 or 2,
   **characterized in that**
   the surfactant substance is an anionic surfactant.

4. Coating lacquer according to at least one of Claims 1 to 3, **characterized in that**
   the surfactant substance is a surfactant based on a sulphosuccinic salt.

5. Coating lacquer according to at least one of the preceding claims, **characterized in that** at least one polar solvent is used as solvent.

6. Coating lacquer according to at least one of the preceding claims, **characterized in that**
   alcohol is used as solvent.

7. Coating lacquer according to at least one of the preceding claims, **characterized in that**
   water is used as solvent.

8. Coating lacquer according to at least one of the preceding claims, **characterized in that**
   binders used comprise at least one polymer or one copolymer having carboxy, carboxylate, amine, ammonium, amide or alcohol functionalities.

9. Coating lacquer according to at least one of the preceding claims, **characterized in that**
   binders used comprise a water-soluble polymer or a water-soluble copolymer.

10. Coating lacquer according to at least one of the preceding claims, **characterized in that**
    the binder has been selected from the group of polyvinyl alcohol, polyvinyl butyral, polyester resin, maleate resin or cellulose derivative.

11. Coating lacquer according to at least one of the preceding claims, **characterized in that**
    the coating lacquer has a viscosity of from 50 to 500 mPa*s, prior to drying.

12. Coating lacquer according to at least one of the preceding claims, **characterized in that**
    the surface obtained after drying of the coating lacquer has

    - a contact angle with water of at most 23° and
    - a surface tension of at least 65 mN/m.

13. Coating lacquer according to at least one of the preceding claims, **characterized in that**
    the transport velocity of an aqueous test liquid (deionized water and 1% by weight of naphthol red) in a channel (75 $\mu$m $\times$ 1 mm) in which the dried coating lacquer forms a wall of the channel amounts to at least 20 mm/s.

14. Coating lacquer according to at least one of the preceding claims, **characterized in that**
    the transport velocity of an aqueous test liquid (deionized water and 1% by weight of naphthol red) in a channel (75 $\mu$m $\times$ 1 mm) in which the dried coating lacquer forms a wall of the channel amounts to at least 40 mm/s.

15. Coating lacquer according to at least one of the preceding claims, **characterized in that**
    the transport velocity of an aqueous test liquid (deionized water and 1% by weight of naphthol red) in a channel (75 $\mu$m $\times$ 1 mm) in which the dried coating lacquer forms a wall of the channel slows by at most 20% of the initial value after storage for 10 weeks at 70°C.

16. Coating lacquer according to at least one of the preceding claims, **characterized in that**
    the coating lacquer is applied by means of a printing process to a substrate material.

17. Coating lacquer according to at least one of the preceding claims, **characterized in that**
    the coating lacquer is applied by the flexographic printing process to a substrate material.

**18.** Use of the dried coating lacquer according to at least one of the preceding claims as coating in medical applications.

**19.** Use of the dried coating lacquer according to at least one of the preceding claims as coating in diagnostic strips, biosensors, point-of-care devices or microfluidic devices, by means of which biological liquids are investigated.

**20.** Use of the dried coating lacquer according to at least one of the preceding claims as coating in biosensors or analytical test strips, where the arrangement of the material is such that the coating at least partially forms at least one wall of a transport channel or reaction channel.

**Revendications**

**1.** Laque de revêtement constituée par
au moins une substance tensioactive présentant

- une concentration critique de formation de micelles (CMC, 25°C) d'au plus 2,3 g/l et
- une tension superficielle (25°C) de la solution aqueuse à 0,1% en poids d'au plus 50 mN/m,

au moins un solvant,
au moins un liant, ainsi que
le cas échéant d'autres additifs,
où la surface obtenue après le séchage de la laque de revêtement présente

- un angle de contact avec l'eau d'au plus 30° et
- une tension superficielle d'au moins 60 mN/m.

**2.** Laque de revêtement selon la revendication 1, **caractérisée en ce que** la substance tensioactive présente une concentration critique de formation de micelles (CMC, 25°C) d'au plus 1,5 g/l et/ou une tension superficielle de la solution aqueuse à 0,1% en poids d'au plus 45 mN/m.

**3.** Laque de revêtement selon l'une quelconque des revendications 1 ou 2, **caractérisée en ce que** la substance tensioactive est un agent tensioactif anionique.

**4.** Laque de revêtement selon au moins l'une quelconque des revendications 1 à 3, **caractérisée en ce que** la substance tensioactive est un agent tensioactif à base d'un sel de l'acide sulfosuccinique.

**5.** Laque de revêtement selon au moins l'une quelconque des revendications précédentes, **caractérisée en ce qu'**on utilise au moins un solvant polaire comme solvant.

**6.** Laque de revêtement selon au moins l'une quelconque des revendications précédentes, **caractérisée en ce qu'**on utilise un alcool comme solvant.

**7.** Laque de revêtement selon au moins l'une quelconque des revendications précédentes, **caractérisée en ce qu'**on utilise de l'eau comme solvant.

**8.** Laque de revêtement selon au moins l'une quelconque des revendications précédentes, **caractérisée en ce qu'**on utilise comme liant au moins un polymère ou un copolymère présentant des fonctionnalités carboxyle, carboxylate, amine, ammonium, amide ou alcool.

**9.** Laque de revêtement selon au moins l'une quelconque des revendications précédentes, **caractérisée en ce qu'**on utilise comme liant un polymère soluble dans l'eau ou un copolymère soluble dans l'eau.

**10.** Laque de revêtement selon au moins l'une quelconque des revendications précédentes, **caractérisée en ce que** le liant est choisi dans le groupe formé par le poly(alcool vinylique), le polyvinylbutyral, la résine de polyester, la résine de maléinate ou un dérivé de cellulose.

**11.** Laque de revêtement selon au moins l'une quelconque des revendications précédentes, **caractérisée en ce que** la laque de revêtement présente, avant le séchage, une viscosité de 50 à 500 mPa.s.

**12.** Laque de revêtement selon au moins l'une quelconque des revendications précédentes, **caractérisée en ce que** la surface obtenue après le séchage de la laque de revêtement présente

- un angle de contact avec l'eau d'au plus 23° et
- une tension superficielle d'au moins 65 mN/m.

**13.** Laque de revêtement selon au moins l'une quelconque des revendications précédentes, **caractérisée en ce que** la vitesse de transport d'un liquide test aqueux (eau désionisée et 1% en poids d'amarante) dans un canal (75 $\mu$m x 1 mm), dans lequel la laque de revêtement séchée forme une paroi du canal, est d'au moins 20 mm/s.

**14.** Laque de revêtement selon au moins l'une quelconque des revendications précédentes, **caractérisée en ce que** la vitesse de transport d'un liquide test aqueux (eau désionisée et 1% en poids d'amarante) dans un canal (75 $\mu$m x 1 mm), dans lequel la laque de revêtement séchée forme une paroi du canal, est d'au moins 40 mm/s.

**15.** Laque de revêtement selon au moins l'une quelconque des revendications précédentes, **caractérisée en ce que** la vitesse de transport d'un liquide test aqueux (eau désionisée et 1% en poids d'amarante) dans un canal (75 $\mu$m x 1 mm), dans lequel la laque de revêtement séchée forme une paroi du canal, est ralentie, après un entreposage de 10 semaines à 70°C, d'au maximum 20% de la valeur de départ.

**16.** Laque de revêtement selon au moins l'une quelconque des revendications précédentes, **caractérisée en ce que** la laque de revêtement est appliquée au moyen d'un procédé d'impression sur un matériau support.

**17.** Laque de revêtement selon au moins l'une quelconque des revendications précédentes, **caractérisée en ce que** la laque de revêtement est appliquée au moyen d'un procédé d'impression flexo sur un matériau support.

**18.** Utilisation de la laque de revêtement séchée selon au moins l'une quelconque des revendications précédentes comme revêtement dans des applications médicales.

**19.** Utilisation de la laque de revêtement séchée selon au moins l'une quelconque des revendications précédentes comme revêtement dans des bandelettes diagnostiques, des biocapteurs, des dispositifs de point de soin ou de dispositifs microfluidiques au moyen desquel(le)s des liquides biologiques sont analysés.

**20.** Utilisation de la laque de revêtement séchée selon au moins l'une quelconque des revendications précédentes comme revêtement dans des biocapteurs ou des bandelettes test analytiques, où le matériau est disposé de manière telle que le revêtement forme au moins partiellement au moins une paroi d'un canal de transport ou de réaction.

Fig. 1a

Fig. 1b

Fig. 2

Fig. 3a

Fig. 3b

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20020112961 A1
- US 6601613 B2
- US 7125711 B2
- EP 1525916 A1
- DE 10234564 A1
- US 5759364 A1
- WO 2005033698 A1
- US 5997817 A1
- WO 200511160 A1
- US 6955738 B2
- WO 0102093 A2
- DE 3021166 A1
- DE 19849008 A1
- EP 1358896 A1
- WO 0167099 A1
- DE 19815684 A1
- US 20050084681 A1
- EP 1647568 A1
- US 20020110486 A1
- EP 1394535 A1
- US 6969166 B2


**In der Beschreibung aufgeführte Nicht-Patentliteratur**

- Technische Strömungslehre. **W. Bohl.** Technische Strömungslehre. Vogel Verlag, Juni 2005, vol. 13, 37
- Die Tenside. Kosswig/Stache. Carl Hanser Verlag, 1993, 23
- Ullmann's Encyclopedia of Industrial Chemistry. 1994, vol. A25, 747
- Untersuchung über die Wechselwirkung Polymer - Tensid. **VON POPESCU et al.** Colloid & Polymer Science. Springer Verlag, 1972, vol. 250, 303f
- Plastics Additives Handbook. Carl Hanser Verlag